# EUROPEAN PATENT SPECIFICATION

(11) **EP 2 531 857 B1**
(45) Date of publication and mention of the grant of the patent: **18.01.2017**
(21) Application number: 11702698.9
(22) Date of filing: 04.02.2011
(51) Int. Cl.: G01N 33/574, G01N 33/68

(54) **INFECTION PROGNOSTIC ASSAY**
INFEKTIONSPROGNOSETEST
ESSAI DE PRONOSTIC D'INFECTION

(30) Priority: 05.02.2010 GB 201001950
(43) Date of publication of application: 12.12.2012
(73) Proprietor: The Binding Site Group Limited, Birmingham B15 1QT (GB)
(72) Inventor: MEAD, Graham, Birmingham, B15 1QT (GB); BRADWELL, Arthur, Birmingham, B15 1QT (GB)
(74) Representative: Elsy, David
(86) International application number: PCT/GB2011/050197
(87) International publication number: WO 2011/095820

(56) References cited:
- US-A- 4 792 529
- MARSHALL G ET AL: "Borderline high serum free light chain [kappa]/[lambda] ratios are seen not only in dialysis patients but also in non-dialysis-dependent renal impairment and inflammatory states", AMERICAN JOURNAL OF CLINICAL PATHOLOGY 2009 AMERICAN SOCIETY OF CLINICAL PATHOLOGISTS USA LNKD- DOI:10.1309/AJCP8VOT5TVLAQBQ,, vol. 132, no. 2, 1 August 2009 (2009-08-01), page 309, XP002625348,
- FAGNART O C ET AL: "FREE KAPPA AND LAMBDA LIGHT CHAIN LEVELS IN THE CEREBROSPINAL FLUID OF PATIENTS WITH MULTIPLE SCLEROSIS AND OTHER NEUROLOGICAL DISEASES", JOURNAL OF NEUROIMMUNOLOGY, ELSEVIER SCIENCE PUBLISHERS BV, XX, vol. 19, no. 1-2, 1 January 1988 (1988-01-01), pages 119-132, XP002266256, ISSN: 0165-5728, DOI: DOI:10.1016/0165-5728(88)90041-0
- BALMER PAUL ET AL: "Anti-pneumococcal antibody titre measurement: what useful information does it yield?", JOURNAL OF CLINICAL PATHOLOGY APR 2007, vol. 60, no. 4, April 2007 (2007-04), pages 345-350, ISSN: 0021-9746

## Description

The invention relates to a method of prognosis of a human patient with an infection, and/or identifying a human patient at greater risk from an infection. The infection is a bacterial infection selected from, a lower respiratory tract infection (such as pneumonia), urinary tract infection, *Clostridium difficile* infection, septicaemia or peritonitis.

The Applicants have for many years studied free light chains as a way of assaying for a wide-range of monoclonal gammopathies in patients. The use of such free light chains in diagnosis is reviewed in detail in the book "Serum Free Light Chain Analysis, Fifth Edition (2008) A.R. Bradwell et al, ISBN 0704427028".

Antibodies comprise heavy chains and light chains. They usually have a two-fold symmetry and are composed of two identical heavy chains and two identical light chains, each containing variable and constant region domains. The variable domains of each light-chain/heavy-chain pair combine to form an antigen-binding site, so that both chains contribute to the antigen-binding specificity of the antibody molecule. Light chains are of two types, κ and λ and any given antibody molecule is produced with either light chain but never both. There are approximately twice as many κ as λ molecules produced in humans, but this is different in some mammals. Usually the light chains are attached to heavy chains. However, some unattached "free light chains" are detectable in the serum or urine of individuals. Free light chains may be specifically identified by raising antibodies against the surface of the free light chain that is normally hidden by the binding of the light chain to the heavy chain. In free light chains (FLC) this surface is exposed, allowing it to be detected immunologically. Commercially available kits for the detection of κ or λ free light chains include, for example, "Freelite™", manufactured by The Binding Site Limited, Birmingham, United Kingdom. The Applicants have previously identified that determining the amount of free κ/free λ ratios, aids the diagnosis of monoclonal gammopathies in patients. It has been used, for example, as an aid in the diagnosis of intact immunoglobulin multiple myeloma (MM), light chain MM, non-secretory MM, AL amyloidosis, light chain deposition disease, smouldering MM, plasmacytoma and MGUS (monoclonal gammopathies of undetermined significance). Detection of FLC has also been used, for example, as an aid to the diagnosis of other B-cell dyscrasia and indeed as an alternative to urinary Bence Jones protein analysis for the diagnosis of monoclonal gammopathies in general.

Conventionally, an increase in one of the λ or κ light chains and a consequently abnormal ratio is looked for. For example, multiple myelomas result from the monoclonal multiplication of a malignant plasma cell, resulting in an increase in a single type of cell producing a single type of immunoglobulin. This results in an increase in the amount of free light chain, either λ or κ, observed within an individual. This increase in concentration may be determined, and usually the ratio of the free κ to free λ is determined and compared with the normal range. This aids in the diagnosis of monoclonal disease. Moreover, the free light chain assays may also be used for the following of treatment of the disease in patients. Prognosis of, for example, patients after treatment for AL amyloidosis may be carried out.

Katzman et al (Clin. Chem. (2002); 48(9): 1437-1944) discuss serum reference intervals and diagnostic ranges for free κ and free λ immunoglobulins in the diagnosis of monoclonal gammopathies. Individuals from 21-90 years of age were studied by immunoassay and compared to results obtained by immunofixation to optimise the immunoassay for the detection of monoclonal free light chains (FLC) in individuals with B-cell dyscrasia.
The amount of κ and λ FLC and the κ/λ ratios were recorded allowing a reference interval to be determined for the detection of B-cell dyscrasias.

Bacterial infections are a major cause of death in both the general population and those with kidney disease. There are many reasons why individuals with kidney disease are at increased risk of infection, one of which is the retention of uraemic toxins. Uraemic toxins are molecules that in health are removed by the kidney but in kidney failure their serum concentrations increase. An Austrian research team has published studies reporting that FLCs have a negative effect on neutrophil function (For review see Cohen & Horl "Free immunoglobulin light chains as a risk factor in renal and extrarenal complications" (2009) Seminars in Dialysis; 22: 369-372). Neutrophils form only one part of the immune defence system, all the reported studies have been *in vitro* and no causative effects have been demonstrated *in vivo.* However, the Applicant realised that with the possibility of FLCs influencing neutrophil function and the known association of raised FLCs with inflammatory processes, they hypothesised that by measuring serum FLCs, patients at risk of infection-related mortality might be identified.

Marshall et al. (Am J Clin Pathol 2009; 132:308-309) comments on the high serum free light chain (kappa/lambda) ratios, but not amounts, of FLC seen in patients with renal impairment, infection, gangrene, chronic liver disease, malignancy, colitis and vasculitis. Marshall et al. did not specifically comment on any link between the amounts or even ratios of FLC. The fact that no correlation was reported suggests that none was found, suggesting that there is no reason to measure FLC concentrations in patients with infections.

US4792529 discloses the increase of kappa FLC in cerebrospinal fluid (CSF) in patients with multiple sclerosis (MS). Furthermore, US4792529 reports that 38% of the samples from patients with various CNS inflammatory and infectious diseases exhibit abnormal levels of lambda FLC, but not kappa FLC. A selective increase in the CSF indicates localised production of the light chains. Localised immunoglobulin production in the CNS is not commonplace, and so normal levels of FLC and intact immunoglobulins are low in normal CSF. Localised production of light chains in the CSF may therefore result in a discernable increase in FLC levels. In contrast, immunoglobulin levels in normal serum are found in g/L quantities. Here, the antibody levels are systemic and not due to localised production. In the case of an infection, antibodies specific for a particular antigen may be produced, but these antibodies would only represent less than 1% of the immunoglobulin pool. This increase in FLC is not expected to be discernable from the background production of FLC. US4792529 further states that although CSF kappa light chain levels were significantly higher in MS patients than controls, no significant differences in serum levels were seen between groups. Therefore, US4792529 teaches that assaying for an increase in systemic FLC is not likely to indicate the presence of a bacterial infection.

Fagnart et al. (Journal of Neuroimmunology, 1988, 19:119-132) also reports a correlation between kappa FLC and MS in CSF. A number of viral and bacterial diseases of the CNS are studied, but there are large discrepancies between different infections and whether CNS or sera was tested. Mean free kappa in sera did not vary between the control and test groups, although the paper does highlight an increase in lambda FLC serum in the control groups. However, this observation was shown for both sera from patients with infectious and non-infectious neurological disorders, and the increase was higher in those patients with non-infectious neurological disorders. Finally, this paper concludes that identifying a correlation between local production of FLC in the CNS can be seen in MS only, similar to US4792529.

The Applicants have now identified that assaying for FLC and especially total FLC can be used in a method of prognosis for a subject with an infection, identifying a subject at greater risk from an infection and/or identifying a subject at risk of developing an infection. They have found that FLC concentration is statistically significantly linked to risk of death in subjects from infection and to be indicative of the presence of such an infection.

The concentration of FLC in serum from individuals that are apparently healthy is influenced to some extent by the ability of the individual's kidneys to filter and excrete FLC. In individuals where FLC clearance is restricted, there is an increase in the levels of FLC found in serum. As a consequence, it is now believed that FLC is a good marker of renal function. Because monomeric FLC kappa molecules (25kDa) are of different size to dimeric lambda molecules (50kDa), together they are better markers of glomerular filtration than, for example, creatinine 113 kDa). However, in contrast to creatinine, production of FLCs may result as a consequence of many diseases, so serum FLCs will typically not be used as a renal function marker, in isolation.

However, markers of B-cell proliferation/activity are important and because B-cells are responsible for making FLCs, this is clinically useful. FLC production is an early indicator of B-cell up-regulation. In this respect it can complement the use of CRP which is a T-cell mediated marker of inflammatory responses.

High FLC concentrations may well be an indication of chronic renal or inflammatory disorders or B-cell dyscrasias. Hence, an abnormal FLC assay result may be a marker of a variety of disorders that currently require several tests in combination. The converse of this, when the FLC assay results are normal, indicates good renal function, no inflammatory conditions and no evidence of B-cell dyscrasia.

The Applicant studied serum samples from patients having various degrees of renal impairment. The causes of death of patients was investigated in comparison with FLC concentration. FLC levels, and especially total FLC levels, were observed to be clearly associated with risk of death from infection.

The invention provides a method of prognosis of a human patient with an infection, and/or identifying a human patient at greater risk of death from an infection, the method comprising detecting an amount of free light chains (FLC) in a sample of blood or serum from the human patient, wherein a higher amount of FLC is associated with decreased survival due to the infection and/or increased risk from an infection wherein the infection is a bacterial infection selected from a lower respiratory tract infection (such as pneumonia), urinary tract infection, *Clostridium difficile* infection, septicaemia or peritonitis.

The subject may not have been previously diagnosed with an infection. The FLC may be kappa or lambda FLC. However, preferably the total FLC concentration is measured, as detecting kappa FLC or lambda FLC alone may miss, for example, abnormally high levels of one or other FLC produced for example monoclonally in the patient.

Total free light chain means the total amount of free kappa plus free lambda light chains in a sample.

Preferably the subject does not necessarily have symptoms of a B-cell associated disease. The symptoms may include recurrent infections, bone pain and fatigue. Such a B-cell associated disease is preferably not a myeloma, (such as intact immunoglobulin myeloma, light chain myeloma, non-secretory myeloma), an MGUS, AL amyloidosis, Waldenström's macroglobulinaemia, Hodgkin's lymphoma, follicular centre cell lymphoma, chronic lymphocytic leukaemia, mantle cell lymphoma, pre-B cell leukaemia or acute lymphoblastic leukaemia. Moreover, the individual typically does not have reduced bone marrow function. The individual typically does not have an abnormal κ : λ FLC ratio, typically found in many such diseases.

The term "total free light chains" means the amount of κ and λ free light chains in the sample from the subject.

Typically the FLC, such as total FLC, is determined by immunoassay, such as ELISA assays, Serum Protein Electrophoresis (SPE), or utilising fluorescently labelled beads, such as Luminex ™ beads.

Total free light chain means the total amount of free kappa plus free lambda light chains in a sample.

Preferably the subject does not necessarily have symptoms of a B-cell associated disease. The symptoms may include recurrent infections, bone pain and fatigue. Such a B-cell associated disease is preferably not a myeloma, (such as intact immunoglobulin myeloma, light chain myeloma, non-secretory myeloma), an MGUS, AL amyloidosis, Waldenstrom's Sandwich assays, for example use antibodies to detect specific antigens. One or more of the antibodies used in the assay may be labelled with an enzyme capable of converting a substrate into a detectable analyte. Such enzymes include horseradish peroxidase, alkaline phosphatase and other enzymes known in the art. Alternatively, other detectable tags or labels may be used instead of, or together with, the enzymes. These include radioisotopes, a wide range of coloured and fluorescent labels known in the art, including fluorescein, Alexa fluor, Oregon Green, BODIPY, rhodamine red, Cascade Blue, Marina Blue, Pacific Blue, Cascade Yellow, gold; and conjugates such as biotin (available from, for example, Invitrogen Ltd, United Kingdom). Dye sols, chemiluminescent labels, metallic sols or coloured latex may also be used. One or more of these labels may be used in the ELISA assays according to the various inventions described herein or alternatively in the other assays, labelled antibodies or kits described herein.

The construction of sandwich-type assays is itself well known in the art. For example, a "capture antibody" specific for the FLC is immobilised on a substrate. The "capture antibody" may be immobilised onto the substrate by methods which are well known in the art. FLC in the sample are bound by the "capture antibody" which binds the FLC to the substrate via the "capture antibody".

Unbound immunoglobulins may be washed away.

In ELISA or sandwich assays the presence of bound immunoglobulins may be determined by using a labeled "detecting antibody" specific to a different part of the FLC of interest than the binding antibody.

Flow cytometry may be used to detect the binding of the FLC of interest. This technique is well known in the art for, e.g. cell sorting. However, it can also be used to detect labeled particles, such as beads, and to measure their size. Numerous text books describe flow cytometry, such as Practical Flow Cytometry, 3rd Ed. (1994), H. Shapiro, Alan R. Liss, New York, and Flow Cytometry, First Principles (2nd Ed.) 2001, A.L. Given, Wiley Liss.

One of the binding antibodies, such as the antibody specific for FLC, is bound to a bead, such as a polystyrene or latex bead. The beads are mixed with the sample and the second detecting antibody. The detecting antibody is preferably labeled with a detectable label, which binds the FLC to be detected in the sample. This results in a labeled bead when the FLC to be assayed is present.

Other antibodies specific for other analytes described herein may also be used to allow the detection of those analytes.

Labeled beads may then be detected via flow cytometry. Different labels, such as different fluorescent labels may be used for, for example, the anti- free λ and anti- free κ antibodies. Other antibodies specific for other analytes, such as bacterial-specific antigens, described herein may also be used in this or other assays described herein to allow the detection of those analytes. This allows the amount of each type of FLC bound to be determined simultaneously or the presence of other analytes to be determined.

Alternatively, or additionally, different sized beads may be used for different antibodies, for example for different marker specific antibodies. Flow cytometry can distinguish between different sized beads and hence can rapidly determine the amount of each FLC or other analyte in a sample.

An alternative method uses the antibodies bound to, for example, fluorescently labeled beads such as commercially available Luminex™ beads. Different beads are used with different antibodies. Different beads are labeled with different fluorophore mixtures, thus allowing different analytes to be determined by the fluorescent wavelength. Luminex beads are available from Luminex Corporation, Austin, Texas, United States of America.

Preferably the assay used is a nephelometric or turbidimetric method. Nephelometric and turbidimetric assays for the detection of λ - or κ - FLC are generally known in the art, but not for total FLC assays. They have the best level of sensitivity for the assay. λ and κ FLC concentrations may be separately determined or a single assay for total FLC arrived at. Such an assay contains anti-K and anti-λ FLC antibodies typically at a 60:40 ratio, but other ratios, such as 50:50 may be used.

Antibodies may also be raised against a mixture of free λ and free κ light chains.

The amount of FLC such as total FLC may be compared to a standard, predetermined value to determine whether the total amount is higher or lower than a normal range of FLC.

As discussed in detail below, the Applicants have identified that higher concentrations of serum FLC are associated with a significant increase in the likelihood of reduced survival in patients with infections. More so than, for example, for people with lower serum FLC levels.

A level of >1.7 mg/L of FLC per unit GFR was associated with an increased risk of death from infection. Patients with a level above the 90^{th} percentile (6.12mg/L of FLC per unit GFR) had a very significantly increased risk.

ICD10 levels have shown increased mortality at 50mg/ml and 60mg/ml FLC as measured by SPE.

Historically, assay kits have been produced for measurement of kappa and lambda FLC separately, to allow the calculation of a ratio. They have been conventionally used in individuals already exhibiting disease symptoms.

Preferably the assay is capable of determining FLC, for example total FLC, in the sample for example from approximately 1 mg/L to 100 mg/L, or 1 mg/L - 80 mg/L. This is expected to detect the serum FLC concentrations in the vast majority of individuals without the requirement for re-assaying samples at a different dilution.

Preferably the method comprises detecting the amount of total free light chain in the sample utilising an immunoassay, for example, by utilising a mixture of anti-free κ light chain and anti-free λ light chain antibodies or fragments thereof. Such antibodies may be in a ratio of 50:50 anti-K: anti-λ antibodies. Antibodies, or fragments, bound to FLC may be detected directly by using labelled antibodies or fragments, or indirectly using labelled antibodies against the anti-free λ or anti-free κ antibodies.

The antibodies may be polyclonal or monoclonal. Polyclonal may be used because they allow for some variability between light chains of the same type to be detected as they are raised against different parts of the same chain. The production of polyclonal antibodies is described, for example in WO97/17372.

Preferably, the amount of serum FLC, such as total FLC, identified, and found to be significant to show an increased likelihood of overall survival, is below 50 mg/L. A level of <47.4mg/L showed P<0.001.

Individuals with a corrected FLC level above the median (1.7mg/L FLC per unit GFR-glomerular filtration rate) had a markedly shorter survival. Patients with a level above the 90^{th} percentile (6.12mg/L FLC per unit GFR) had a very significantly increased risk.

The invention will now be described by way of example only, with reference to the following figures:
**Figure 1** shows the probability of survival for a study population divided into quintiles on the basis of their total FLC concentrations. The quintile levels were <33.3, 33.4-47.3, 47.4-76.8, 67.9-106.3 and >106.5 mg/L.
**Figure 2** shows total FLC was corrected for GFR and then separated into quintiles. High total corrected FLC quintiles (4 and 5= lower line) predicted death from severe infections (P<0.001):

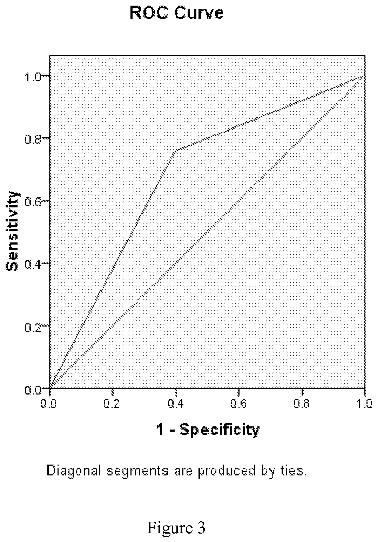

**Figure 3** ROC curve for the two top quintiles as a risk marker for death by infection (corrected level of total FLCs >1.7 mg/1unitGFR considered abnormal).
**Figure 4** is a comparison between the total FLC concentrations obtained using separate, commercially available, anti-free κ and anti-free λ assay kits, compared to a total FLC assay kit using combined anti-λ and anti-K free light chain antibodies.
**Figure 5** shows a comparison of patient deaths over a 4.5 year study for two different FLC concentrations as prognostic cut off points, the table showing causes of death.

### Infection prognosis

### Methods

1300 patients with various degrees of renal impairment had serum samples collected ("Baseline") and were then followed up for a period of up to 63 months.

In more detail the patients were recruited from the renal clinics at the University Hospital Birmingham. The patients had a range of renal problems including proteinuria, haematuria, chronic kidney disease (all stages), end stage renal failure (haemodialysis and peritoneal dialysis) and renal transplant recipients.

The tests and assessments made included:
Serum creatinine and an estimated glomerular filtration rate (eGFR).
A corrected level ofFLCs per unit GFR was calculated as follows: total serum FLC concentration (mg/L) was divided by estimated glomerular filtration rate as calculated by the Cockcroft-Gault equation (REF) in mls/min/1.73m2. Thus giving a serum total FLC level for the patient, independent of renal function, in mg/L per unit GFR. Ref:
   Cockcroft DW, Gault MH: Prediction of creatinine clearance
   from serum creatinine. Nephron 16: 31-41, 1976.
   Urinary albumin/creatinine ratio.
   Serum FLC concentrations, both kappa and lambda (Freelite, The Binding Site, Birmingham, UK).
   Total, serum FLC concentrations were calculated by adding the values for kappa FLC and lambda FLC.

### Follow-up:

Patients were followed up for time to death and cause of death.

### Results

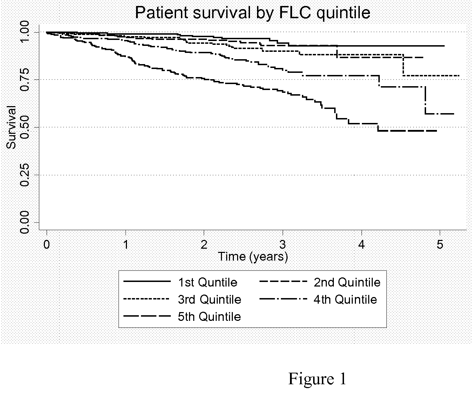

Kaplan Meier analysis of patient survival demonstrated that patients with higher FLC levels had a reduced survival (P<0.001), see Figure 1.

When causes of death were investigated the chances of death secondary to infections was significantly higher in patients with higher total FLC concentrations.

| | | Total FLC Quintiles (n=number of deaths in each group) | | | | | |
|---|---|---|---|---|---|---|---|
| | | 1 | 2 | 3 | 4 | 5 | Total |
| Cause of death | Cardiovascular | 1 | 2 | 2 | 12 | 27 | 44 |
| | Infection | 3 | 1 | 7 | 8 | 19 | 38 |
| | Renal | 0 | 0 | 1 | 4 | 12 | 17 |
| | Cancer | 2 | 2 | 2 | 3 | 7 | 16 |
| | Other | 2 | 0 | 0 | 3 | 1 | 6 |
| | Unknown | 0 | 0 | 0 | 0 | 1 | 1 |
| | Total | 8 | 5 | 12 | 30 | 67 | 122 |

The risk of death from infections was also associated with renal function (CKD stages 1-5), although this did not as clearly delineate an at risk group:

| | | CKD stages (n= number of deaths in each group) | | | | | |
|---|---|---|---|---|---|---|---|
| | | 1.00 | 2.00 | 3.00 | 4.00 | 5.00 | Total |
| Cause.simp | Cardiovascular | 1 | 0 | 15 | 18 | 10 | 44 |
| | Infection | 0 | 2 | 14 | 11 | 11 | 38 |
| | Renal | 0 | 0 | 1 | 8 | 8 | 17 |
| | Cancer | 0 | 2 | 8 | 5 | 1 | 16 |
| | Other | 0 | 1 | 2 | 1 | 2 | 6 |
| | Unknown | 0 | 0 | 0 | 0 | 1 | 1 |
| | Total | 1 | 5 | 40 | 43 | 33 | 122 |

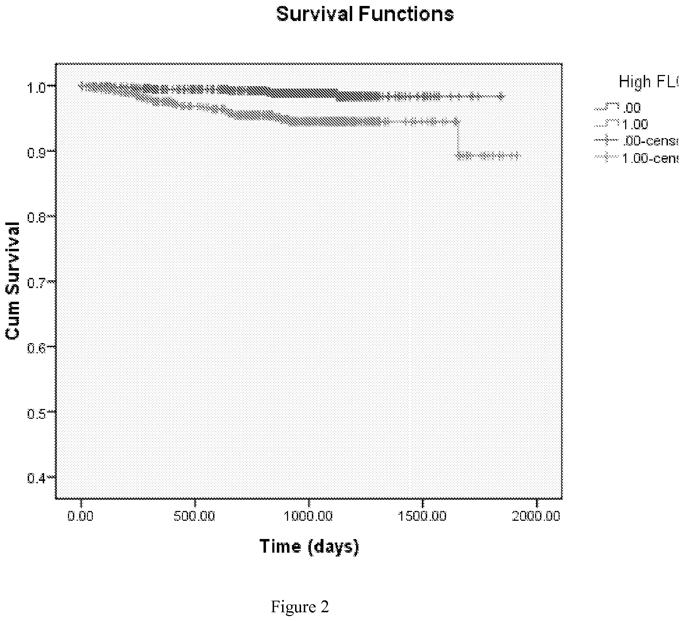

Total FLC and renal function (eGFR) are correlated closely (R-0.645, P<0.001). Therefore to exclude any influence of renal function on the utility of total FLCs to predict infections, total FLC was corrected for GFR and then separated into quintiles. High total corrected FLC quintiles (4 and 5= lower line, Figure 2) predicted death from severe infections (P<0.001):

When these two top quintiles were used as a risk marker for death by infection (corrected level of total FLCs >1.7 mg/1unit GFR considered abnormal) and analysed using a ROC curve the test was significantly predictive (P<0.0001):

The principal infections identified were lower respiratory tract infections (pneumonia), urinary tract infections, *Clostridium difficile,* septicaemia and peritonitis.

### Discussion

The results were gathered in patients with some form of renal impairment. But given that the relationship between elevations of total FLC and infections was independent of renal function it is likely total FLC may be similarly predictive of infections in the general population. It might be speculated that the test(s) will be more sensitive in the general population where there is no background elevation of FLC concentrations due to reduced renal function. As mentioned earlier, studies reporting a suppression of neutrophil function mediated by FLC have previously been published. Neutrophils form only one part of the immune defence system, however, and it has not, to our knowledge, been proposed that measurement of serum FLC concentrations could predict subjects at greater risk of death due to bacterial infection. Cohen and co-workers first reported on the in vitro effect of FLC on leukocytes in 1995 but have not published any studies measuring in vivo FLC concentrations in the period since then. The size of the predictive effect we observed was unexpected, especially after serum FLC concentrations had been corrected for renal function.

### Assay Kit

The method according to the invention may utilise the following assay kit. The assay kit quantifies the total free κ plus free λ light chains present within patient samples, for example, in serum. This may be achieved by coating 100 nm carboxyl modified latex particles with a 50:50 blend of anti-free κ and anti-free λ light chain sheep antibody. In the assay exemplified below, the measuring range for the total free light chains is for 1-80 mg/L. However, other measuring ranges could equally be considered.

Anti-free κ and anti-free λ anti sera are produced using techniques generally known in the art, in this particular case in sheep. The general immunisation process is described in WO 97/17372.

Anti-κ and anti-λ antisera were diluted to equal concentrations using phosphate buffered saline (PBS). Those antibodies were combined to produce antisera comprising 50% anti κ antibody and 50% anti λ antibody.

Antibodies were coated onto carboxyl modified latex at a coat load of 10 mg/lot. This was achieved using standard procedures. See, for example, "Microparticle Reagent Optimization: A laboratory reference manual from the authority on microparticles" Eds: Caryl Griffin, Jim Sutor, Bruce Shull. Copyright Seradyn Inc, 1994 (P/N 0347835(1294).

This reference also provides details of optimising the assay kits using polyethylene glycol (PEG).

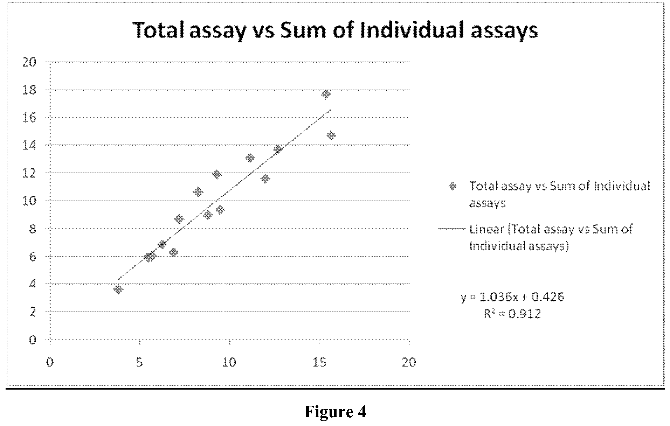

The combined antibodies were compared to results obtained using commercially available κ and λ Freelite™ kits (obtained from the Binding Site Group Limited, Birmingham, United Kingdom). Such Freelite™ kits identify the amount of κ and the amount of λ free light chains in separate assays. The total FLC kits were used to generate curves, which were validated using controlled concentrations. Calibration curves were able to be obtained between 1 and 80 mg/l for total free light chain. In the results table below, results were obtained for κ free light chain (KFLC), λ free light chain (LFLC) and total FLC, using the κ FreeliteTM, λ FreeliteTM and total free light chain assays. These results are shown for 15 different normal serum samples. The results are shown in the table below and in Figure 4 as measured by turbidimetry.

Preliminary results indicate that the principle of using a total free light chain assay based on anti-κ and anti-λ free light chain antibody is viable.

### Results

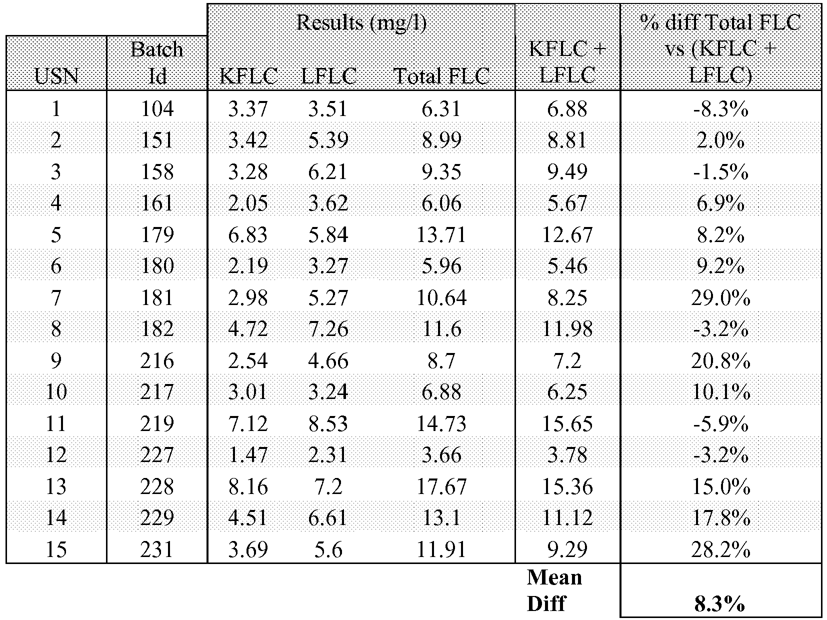

### Further Supporting Data

### Study Population

Between November 8^{th} 2005 and January 10th 2006 the hospital laboratory received 723 serum samples with a request for serum protein electrophoresis (SPE). Samples from paediatric patients, patients on immunoglobulin replacement, second and subsequent samples from the same patient, were excluded from the analysis. Also excluded were all patients with evidence of a monoclonal gammopathy as indicated by an abnormal κ/λ free light chain (FLC) ratio (<0.25 or >1.65; Katzmann) or abnormal SPE result (if confirmed by immunofixation). This left 528 patients in the final data analysis.

### Laboratory Analyses

All sera were analysed for serum protein abnormalities by serum protein electrophoresis (SPE; Sebia, UK). Serum immunofixation (IFE; Sebia) was performed on all samples with the presence of an abnormal SPE band or those with a high index of suspicion (unexplained hypogammaglobulinaemia, broad beta region, or low immunoglobulins with supporting clinical details). As part of an evaluation of serum free light chain (sFLC) analysis in a diagnostic setting, sFLC measurements (Freelite, The Binding Site, Birmingham, UK) were made for all sera using a Siemens Dade-Behring Prospec Nephelometer, in accordance with the manufacturer's instructions.

### Patient Follow-up

In July 2010, 4 years and 6 months after the end of the original study, patient records were reviewed. For all patients, the date of the last follow-up or date of death was recorded and death certificates were obtained. The primary causes of death listed on the death certificates were categorised according to WHO International Statistical Classification of Diseases and Related Health Problems 10th Revision (ICD-10).

### Data Analysis

The association of high combined (κ + λ) FLC concentrations with increased probability of death was investigated using Kaplan Meier survival analysis, Cox regression analysis and Chi-squared analysis. Categorical cut-offs of both > or <50mg/L and > or <65mg/L were investigated.

### Results

Over the 4.5 years of follow-up there were 99 deaths (=18.8% mortality). Higher concentrations of polyclonal serum free light chains (kappa plus lambda) were found to be associated with increased mortality. This was the case if the prognostic cut-off utilised was 50mg/L or 65mg/L and was seen with all statistical analyses. The predominant causes of death were "Circulatory" (cardio-vascular disease) and "Infections/respiratory".

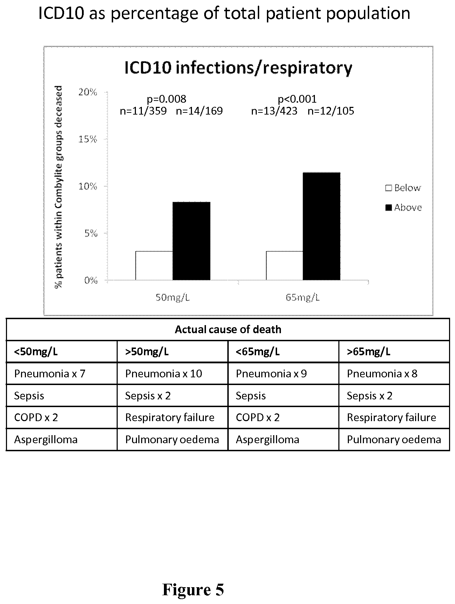

The figure (Figure 5) represents the % of deaths due to infections/respiratory causes in the different categories. Statistical significance was determined by Chi-squared analysis.

## Claims

1. A method of prognosis of a human patient with an infection, and/or identifying a human patient at greater risk of death from an infection, the method comprising detecting an amount of free light chains (FLC) in a sample of blood or serum from the human patient, wherein a higher amount of FLC is associated with decreased survival due to the infection and/or increased risk from an infection, wherein the infection is a bacterial infection selected from a lower respiratory tract infection (such as pneumonia), urinary tract infection, *Clostridium difficile* infection, septicaemia or peritonitis.

2. A method according to claim 1, wherein the total FLC is determined by immunoassay using anti-free light chain antibodies.

3. A method according to claim 2, wherein the antibodies are a mixture of anti-free κ light chain and anti-free λ light chain antibodies.

4. A method according to claim 1, wherein the amount of free light chains is the amount of total free light chains in the sample.

5. A method according to claims 1 to 4, wherein the method comprises detecting the amount of FLC by nephelometry or turbidimetry.

6. A method according to claims 1 to 5, wherein the human patient does not have symptoms of a B-cell associated disease.

## Patentansprüche

1. Verfahren zur Prognose eines Humanpatienten mit einer Infektion und/oder zum Identifizieren eines Patienten mit höherem Todesrisiko durch eine Infektion, wobei das Verfahren ein Erfassen einer Menge an freien Leichtketten (FLC) in einer Blut- oder Serumprobe des Humanpatienten umfasst, wobei eine höhere Menge an FLC mit einem aufgrund der Infektion verminderten Überleben und/oder einem erhöhten Risiko durch eine Infektion assoziiert ist, wobei die Infektion eine bakterielle Infektion ist, ausgewählt aus einer Infektion der unteren Atemwege (wie etwa Pneumonie), Infektion des Harntraktes, *Clostridium-difficile-*Infektion, Septikämie oder Peritonitis.

2. Verfahren gemäß Anspruch 1, bei dem die Gesamtmenge der FLC durch Immunassay mit Antikörpern gegen freie Leichtketten bestimmt wird.

3. Verfahren gemäß Anspruch 2, bei dem die Antikörper eine Mischung aus Antikörpern gegen freie κ-Leichtketten und Antikörpern gegen freie λ-Leichtketten sind.

4. Verfahren gemäß Anspruch 1, bei dem die Menge derfreien Leichtketten die Menge der gesamten freien Leichtketten in der Probe ist.

5. Verfahren gemäß Anspruch bits 4, bei dem das Verfahren ein Erfassen der Menge der FLC durch Nephelometrie oderTurbidimetrie umfasst.

6. Verfahren gemäß Anspruch 1 bis 5, bei dem der Humanpatient keine Symptome einer B-Zellen-assoziierten Erkrankung aufweist.

## Revendications

1. Procédé de diagnostic d'un patient humain atteint d'une infection et/ou d'identification d'un patient humain ayant un grand risque de décès du fait d'une infection, ce procédé comprenant deux étapes consistant à détecter la quantité de chaînes légères libres (FLC) dans un échantillon de sang ou de sérum de ce patient humain, une quantité élevée de FLC étant associée à une survie diminuée du fait de l'infection et/ou à un risque augmenté d'une infection, l'infection étant une infection bactérienne choisie parmi une infection des voies respiratoires inférieures (telle qu'une pneumonie), une infection des voies urinaires, une infection par *Clostridium difficile,* une septicémie ou une péritonite.

2. Procédé conforme à la revendication 1, selon lequel la quantité de FLC totale est déterminée par un test immunologique utilisant des anticorps anti-chaînes légères libres.

3. Procédé conforme à la revendication 2, selon lequel les anticorps sont un mélange d'anticorps anti-chaînes légères κ et d'anticorps anti-chaînes légères λ.

4. Procédé conforme à la revendication 1, selon lequel la quantité de chaînes légères est la quantité de chaînes légères totale dans l'échantillon.

5. Procédé conforme aux revendications 1 à 4, comprenant une étape consistant à détecter la quantité de FLC par néphélométrie ou par tur-bidimétrie.

6. Procédé conforme aux revendications 1 à 5, selon lequel le patient humain n'a pas de symptômes d'une affection associée aux cellules B.
